Europäisches Patentamt

European Patent Office

Office européen des brevets

(19)

(11) EP 0 466 303 B1

(12) **EUROPEAN PATENT SPECIFICATION**

(45) Date of publication and mention
of the grant of the patent:
**24.07.1996 Bulletin 1996/30**

(51) Int Cl.[6]: **G01N 31/00**, G01N 33/18,
G01N 31/16

(21) Application number: **91303813.9**

(22) Date of filing: **26.04.1991**

(54) **Method and system for continuously monitoring and controlling a process stream for dechlorination residual**

Verfahren und System zur kontinuierlichen Überwachung und Regelung von Entchlorisierungsrückständen in einem Prozesstrom

Procédé et dispositif pour le contrôle en continu des résidus de déchloruration dans un courant de traitement

(84) Designated Contracting States:
**DE GB**

(30) Priority: **09.07.1990 US 549994**
**25.03.1991 US 674244**

(43) Date of publication of application:
**15.01.1992 Bulletin 1992/03**

(73) Proprietor: **WALLACE & TIERNAN, INC.**
**Newark, New Jersey 07101-9976 (US)**

(72) Inventors:
- **van Grouw, Albert, III**
  **North Haledon, New Jersey (US)**
- **Stockinger, Gregory**
  **Pompton Plains, New Jersey (US)**

- **Huebner, Wayne B.**
  **Dover, New Jersey (US)**
- **Stannard, James W.**
  **Basking Ridge, New Jersey (US)**

(74) Representative: **W.P. THOMPSON & CO.**
**Eastcheap House**
**Central Approach**
**Letchworth, Hertfordshire SG6 3DS (GB)**

(56) References cited:
**US-A- 4 364 835**

- **J. WATER POLLUTION CONTROL FED., vol. 57, no. 11, 1985, pp. 1068-1073; R.E. FINGER et al.: 'DEVELOPMENT OF AN ON-LINE ZERO CHLORINE RESIDUAL MEASUREMENT AND CONTROL SYSTEM'**

## Description

### Field of the Invention

This invention relates generally to a method and system for continuously monitoring and controlling a process stream for a dechlorination residual. In particular, the invention relates to a method and system for continuously monitoring and controlling a process stream containing a dechlorination residual by biasing a sample thereof with an analyzing agent to determine the amount of dechlorination residual contained in the process stream. Dechlorination is defined here as the complete or partial removal of any oxidant, including chlorine, by the controlled addition of a reducing agent.

### Background of the Invention

Chlorine is commonly used to disinfect sewage treatment plant process stream and for biofouling control in cooling systems. Because chlorine is such a highly effective disinfecting/oxidizing agent, any chlorine unused in the process is just as effective in destroying aquatic life.

In order to eliminate or substantially remove the residual chlorine from a process stream, a dechlorination agent or device is often used. Typically, sulfur dioxide ($SO_2$) is added to the process stream to react quantitatively with the chlorine residual. If $SO_2$ is added in excess of the amount of chlorine residual in the process stream, the chlorine will be completely eliminated.

The amount of unreacted dissolved $SO_2$ (i.e., dechlorination residual) remaining in the process stream is preferably maintained at low, but positive, concentration to insure chlorine residual removal and minimize oxygen consumed. This sort of treatment not only protects aquatic life, but results in more efficient use of the $Cl_2$ and $SO_2$ reagents added to the process stream. However, there are no completely acceptable methods or systems available for directly monitoring and controlling the amount of dechlorination residual in a continuous process stream to which a chlorine disinfectant/oxidant has been previously added.

One example of a method for determining the concentration of a chemical constituent in a fluid is disclosed in U.S. Patent 2,560,317. The method disclosed is particularly well-suited for detecting the concentration of residual chlorine in a process stream. The method involves the removal of chlorine from a process stream sample stream by passing the sample through an activated carbon bed and thereafter adding chlorine at a variable and determinable rate to compare the relative concentrations of chlorine in that stream to the relative concentration of chlorine in an untreated process sample stream. When the comparison is brought to a zero difference, by adjusting the rate of chlorine addition, the determinable rate at which chlorine is added to the chlorine free sample stream is an exact measure of the amount of chlorine in the process stream. Iodine is identified as a suitable substitute for chlorine when it is added to the chlorine free stream. Although this method allows the amount of residual chlorine to be measured, it is completely silent as to measuring a dechlorination residual in the process stream.

Other examples of methods for measuring and controlling on-line zero chlorine residual are disclosed in Finger, R.E., et al., "Development Of An On-Line Zero Chlorine Residual Measurement And Control System." J. Water Pollution Control Fed., Vol. 57, No. 11, 1068 (1985). Finger et al. recognize that there are no techniques available to directly monitor or control zero chlorine residuals nor are there any continuous analytical techniques available to monitor dechlorination (i.e., dissolved $SO_2$) residuals.

Finger et al. identify a feed-forward system that incorporates an effluent flow signal and a chlorine residual signal which are measured immediately before the dechlorination point. Finger et al. also identify a feedback residual control system that biases the dechlorination sample with large volumes of gaseous chlorine or liquid hypochlorite reagents before the dechlorination sample is analyzed. However, Finger et al. argue that both of these systems are deficient because they depend on precise sample and biasing reagent flows. Sample biasing with chlorine or hypochlorite is also subject to chemical reaction interferences with other contaminants (e.g., ammonia) which may be present in the process stream.

In an attempt to overcome these problems, Finger et al. provide a complex feedback approach wherein a dechlorination effluent is biased with the chlorinated effluent to form a sample that can be measured with conventional chlorine analyzers. The effluent flows are held at a 1:1 ratio with constant-head tanks and the chlorine residual of the dechlorination effluent is electronically calculated from the measured residuals in the chlorine and dechlorinated effluent based on the equation:

$$C_{post} = 2C_{meas} - C_{pre}$$

where:

$C_{post}$ = the chlorine residual of the dechlorinated effluent;

$C_{pre}$ = the chlorine residual of the effluent prior to dechlorination; and

$C_{meas}$ = the chlorine residual of the 1:1 mixture of pre- and post-dechlorination effluents.

The method requires two analyzers to be used to determine the amount of chlorine residual in the dechlorinated effluent. Excessive instrumentation is necessary because the variability of the residual measuring technique must be minimized to ensure control within regulatory limits. The dechlorinated residual is calculated and controlled from the summation output of the two residual analyzers.

All of the systems discussed above are expensive to install and have substantial operational costs associated with maintenance, sample pumping, and reagent costs. These systems are often inherently unstable, producing measurement errors that typically exceed safe regulatory residual limits by several orders of magnitude. This is especially undesirable since dechlorination control continues to be the subject of more stringent government environmental regulations.

Clearly what is needed is a method and system for directly measuring and controlling a continuous process stream for dechlorination residual. The method and system should permit a chlorine residual to be completely eliminated within the process stream while the amount of dechlorination residual is minimized. Monitoring and control should be conducted under conditions where relatively small volumes of the reagent and process sample are used. Moreover, the reagents used should be relatively stable and should not be subject to chemical reaction interferences with other contaminants which may be present within the process stream. The present invention fills that need.

## Summary of the Invention

The invention comprises methods and systems for monitoring and controlling the amount of a disinfectant removing agent in a continuous process stream. The disinfectant removing agent is added in excess to the process stream to reduce or eliminate a disinfectant residual which has previously been added to the process stream.

The methods of the present invention comprise continuously drawing off a sample of the process stream and continuously adding an analyzing agent, different from the disinfectant, to the sample in an amount sufficient to completely react with the residual disinfectant removing agent and leave an unreacted amount of analyzing agent or, in the case of incomplete disinfectant removal, add to the disinfectant residual. A sufficient amount of time is allowed for the analyzing agent to completely react with the residual disinfectant removing agent. Thereafter, the sample is continuously analyzed to determine the amount of unreacted analyzing agent, or analyzing agent plus disinfectant, remaining in the sample. Based on the amount of analyzing agent added, and the amount of unreacted analyzing agent remaining in the sample after reaction, the amount of residual disinfectant removing agent or disinfectant residual is determined. Using the determined amount of residual disinfectant or disinfectant removing agent, the amount of disinfectant removing agent added to the process stream is controlled.

The system(s) of the present invention comprise a means for drawing off a sample of the process stream. Feed means for adding an analyzing agent, different from the disinfectant, to said sample are provided to supply an amount of analyzing agent sufficient to completely react with the residual disinfectant removing agent and leave an unreacted amount of analyzing agent. Analyzer means for analyzing the sample containing added analyzing agent are provided to determine the amount of residual disinfectant removing agent in the sample. Controller means responsive to the analyzer means and the feed means are provided for continuously selectably varying the amount of disinfectant removing agent added to the process stream based on the determined amount of residual disinfectant removing agent.

## Brief Description of the Drawings

Figure 1 is a schematic illustration of an embodiment of the method and system of the invention.

Figure 2 is a schematic illustration of another embodiment of the method and system of the invention.

Figure 3 is a partial cross-sectional view of a gas purger according to an embodiment of the invention.

Figure 4 is a plain view of a residual analyzer assembly used to determine the amount of dechlorination residual in a process sample.

## Detailed Description of the Invention

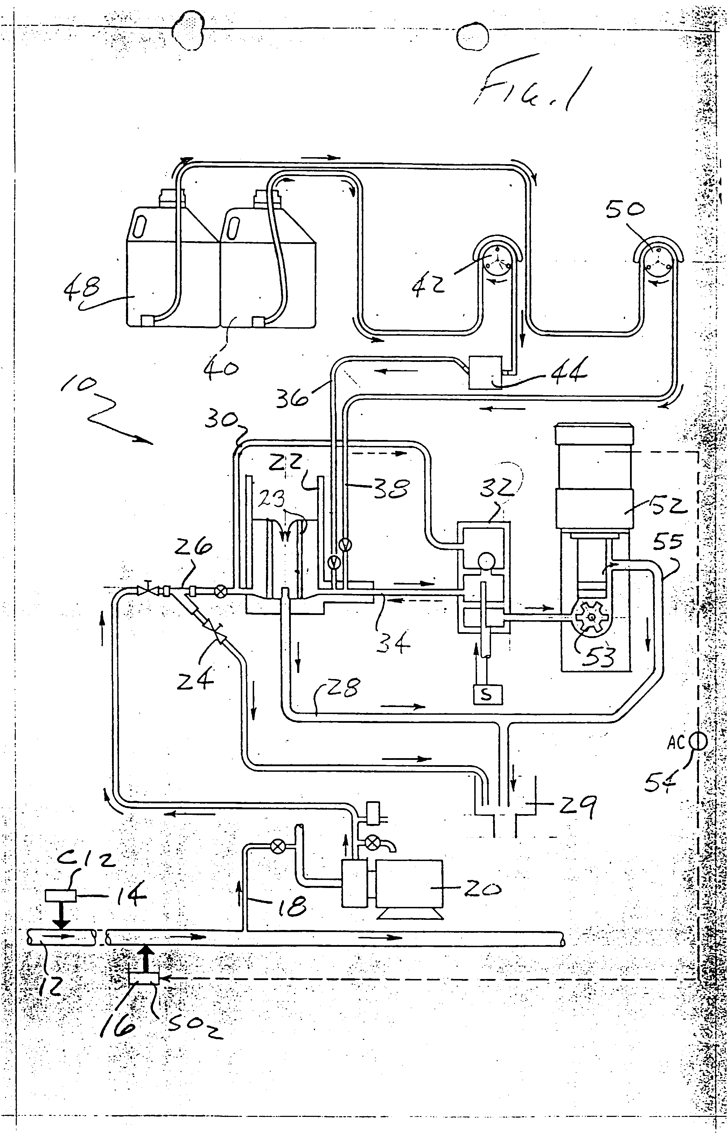

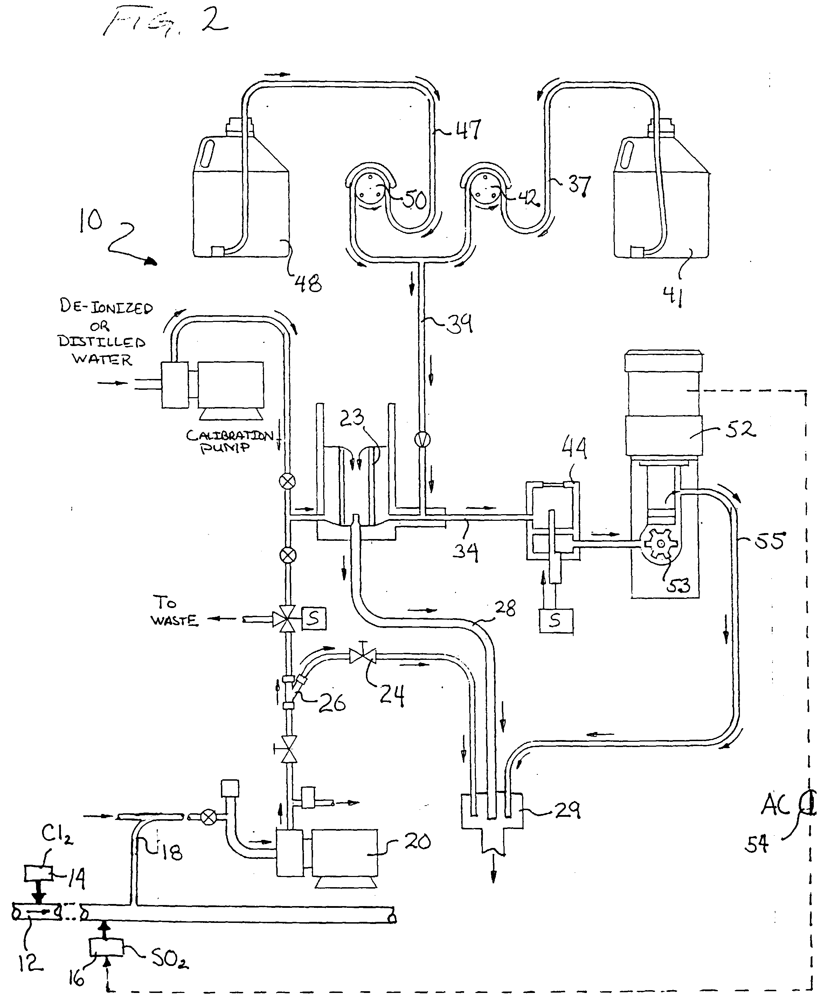

Referring now to the drawings, wherein like numerals indicate like elements, there is shown in each of Figures 1 and 2 a system 10 for monitoring and controlling the amount of dechlorination residual within a process stream 12.

In Figure 1, once the process stream 12 is dechlorinated, the amount of dechlorination residual (i.e., unreacted $SO_2$) is determined by continuously withdrawing a sample stream 18, downstream of the $SO_2$ source 16. The sample is withdrawn by pump 20 and transported to constant level box 22.

Typically, a sample flow of about 1 to 2 gpm will be adequate for monitoring and controlling the amount of dechlorination residual in the process stream. Excess sample is used to clean filter element in Y-fitting 26. Excess sample is removed from system 10 by opening valve 24 just downstream of Y-fitting 26 and by collecting the overflow from constant level box 22.

Excess sample in constant level box 22 pours over internal tube 23 and into stream 28. The excess sample is collected in drain 29 and, preferably, returned to process stream 12. The sample may also be bypassed around constant level box 22 by stream 30 to orifice 32, described in more detail hereinafter.

The constant level box 22 provides a constant head of dechlorinated process sample. The dechlorinated process sample held in constant level box 22 is then passed through stream 34 to orifice 32. In the embodiment illustrated in Figure 1, as the dechlorinated process leaves constant level box 22 under the force of gravity, a continuous stream of an analyzing agent solution 36, different from the process disinfecting agent, is introduced in an amount sufficient to completely react with the dechlorination residual in the dechlorinated sample stream and leave an unreacted amount of analyzing agent.

Any suitable analyzing agent can be employed when practicing the present invention. Examples of suitable analyzing agents include, but are not limited to: iodine, chlorine gas, hypochlorite solutions, and the like and/or mixtures thereof. The presently preferred analyzing agent is iodine. Unlike chlorine gas or hypochlorite solutions, iodine reacts much more slowly with ammonia which is commonly found in sewage treatment plant process stream.

While iodine is the presently preferred analyzing agent, any suitable source of iodine can be employed when practicing the present invention. For example, the iodine can be derived from an iodine source (e.g., item 40 as illustrated in Figure 1), or from a reaction between iodate and iodide. A specific manner in which iodine can be derived from a chemical reaction between an iodate and an iodide will be discussed later.

Referring again to Figure 1, in one embodiment of a preferred practice, an iodine solution stream 36 is drawn from an iodine source 40 and precisely metered by a peristaltic pump 42 in order to introduce the iodine solution into the dechlorinated process sample at a fixed concentration and rate. Based on the concentration and flow rate of the iodine solution, the exact amount of iodine is readily calculated. The iodine solution stream 36 is passed through a gas purger 44 in order to remove any gas bubbles which may be contained within stream 36. As will be described in greater detail later, gas purger 44 contains a hydrophobic membrane 46 for releasing any gas bubbles which may be present in stream 36.

Also, in the embodiment illustrated in Figure 1, a separate buffering solution stream 38 is employed to introduce the buffering solution used in the downstream biasing process into the dechlorinated sample stream. The buffering solution is added to the system via stream 38 to stabilize the pH of the mixture. The buffering solution stream 38 is drawn from a buffering solution source 48 and metered by a peristaltic pump 50 in order to introduce the buffering solution to the dechlorinated process sample at a determinable rate.

Following the addition of the iodine solution and buffering solution to the dechlorinated process sample, a chemical reaction takes place between the dechlorination residual ($SO_2$) and the iodine.

The process sample with $SO_2$ and $I_2$ completely reacted is passed through orifice 32 and into analyzer 52. Orifice 32 acts to control the flow rate of the reaction treated process sample into analyzer 52. The analyzer 52 preferably contains an amperometric cell which allows the amount of unreacted $I_2$ to be determined through direct measurement. Analyzer 52 is described in greater detail hereinafter and is also the subject of U. S. Patent 4,822,474.

Based on the amount of unreacted $I_2$ measured by

analyzer 52, and the known metered amount of $I_2$ added in iodine solution stream 36, the amount of $SO_2$ (i.e., dechlorination residual) within process stream 18 can be calculated. Based on the calculated amount of dechlorinated residual within process sample stream 18, the amount of $SO_2$ introduced upstream in process stream 12, via $SO_2$ introduction means 16, is controlled by controller 54. After the analysis is complete, the analyzed sample is then exhausted to drain 29 through stream 55 for collection and removal from system 10.

Those skilled in the art will recognize that pump 20, constant level box 22, orifice 32, peristaltic pumps 42 and 50, analyzer 52, and controller 54 are all commonly used process equipment. As a result, a detailed description of their structure and operation is not necessary to understanding the method and system of the invention.

Another embodiment of a presently preferred practice is illustrated in Figure 2. As in Figure 1, Figure 2 also illustrates a system 10 for monitoring and controlling the amount of dechlorination residual within a process stream 12, wherein iodine is used as the biasing agent. Three of the major differences between the embodiments illustrated in Figures 1 and 2 are as follows: **(a)** the manner in which the iodine and the buffering solutions are introduced into system 10, **(b)** the origin of the iodine solution and **(c)** the location of the gas purge valve 44.

For example, in Figure 1, the iodine employed in the biasing process originates from iodine source 40. There, the iodine is supplied into process stream 34 via stream 36. Also in Figure 1, the buffering solution is supplied into process stream 34 via stream 38. On the other hand, in Figure 2, both the iodine and the buffering solutions are supplied into process stream 34 through stream 39.

In the specific embodiment illustrated in Figure 2, the iodine employed as the biasing agent is derived from a chemical reaction between an iodate, an iodide and an acidic solution. When practicing this embodiment of the invention, the acidic solution, the iodate and the iodide are metered into a reaction chamber or zone 39. This reaction chamber/zone is located upstream of the point where the resulting iodine solution will enter process stream 34.

One method of introducing the reaction components into the reaction chamber/zone comprises introducing the acidic solution, the iodate and the iodide through separate streams such that the components are not combined with one another until they enter the reaction chamber/zone. Another manner in which these reaction components can be introduced into the reaction chamber/zone comprises introducing the acidic solution through a first stream and introducing a combination of the iodate and iodide solutions through a second stream (e.g., see Figure 2).

If the iodate and iodide components are introduced into the reaction chamber/zone as an iodate/iodide solution, the iodate/iodide solution is preferably maintained at a certain level of alkalinity such that the iodate does not begin to react with the iodide to produce a greater than minimal amount of iodine. Typically, if the iodate and iodide are supplied to the reaction chamber/zone as a common solution, the pH of the iodate/iodide solution should be maintained at a value of at least about 9.5. For even improved stability, the pH of the iodate/iodide solution should be maintained at a value of at least about 10.5, preferably, at a value of at least about 11.5.

Any suitable means can be employed to maintain the iodate/iodide solution at the appropriate level of alkalinity. The presently preferred method is to employ a suitable metal hydroxide which will not adversely affect the biasing process of the present invention. Examples of suitable metal hydroxides which can be employed for this purpose include, but are not limited to: potassium hydroxide, sodium hydroxide, magnesium hydroxide, and the like, and/or any combination thereof. It should be noted that, if the iodate and iodide reaction components are supplied to the reaction chamber/zone via separate and distinct streams such that they do not interact with one another until after entering the reaction zone 39, it is not necessary to maintain a particular level of alkalinity.

In the embodiment illustrated in Figure 2, the iodate and iodide components are supplied as a common solution via iodate/iodide solution source 41. Specifically, in Figure 2, the iodate/iodide solution is metered into stream 39 (which acts as the reaction zone) by peristallic pump 42 via stream 37.

At the same time, the acidic solution is metered from source 48 by peristallic pump 50, along stream 47, also into stream 39. While the acidic buffering solution and the iodate/iodide solution are passing within stream 39 (i.e., the reaction zone), they are reacting with one another to form an iodine-containing reaction product. This reaction product is then fed into sample stream 34 for biasing purposes in accordance with the present invention.

If the embodiment is employed wherein the iodine source is derived from a reaction between an iodate, an iodide and an acidic solution, any suitable iodate can be employed. Examples of suitable iodates include, but are not limited to, metal-iodates, such as potassium iodate, sodium iodate and the like.

Regarding the iodide employed as a reaction component of this latter embodiment, any suitable iodide can be employed which, when in the presence of an acidic environment, will react with the iodate to produce iodine. Examples of suitable iodides include, but are not limited to, metal iodides, such as potassium iodide, and rubidium iodide and the like.

Regarding the acidic solution employed as a reaction component of this latter embodiment, it must be able to react with the iodate and iodide to produce an iodine-containing reaction product. Moreover, it must not adversely affect the operation of the downstream biasing process of the present invention.

Any such suitable acidic solution can be employed when practicing this embodiment of the invention. Typically, organic or inorganic acids can be employed for this purpose. Examples of such suitable acidic solutions include, but are not limited to, acetic acid, sulfuric acid, hydrochloric acid, and the like and/or any combination thereof. The presently preferred buffering agent comprises acetic acid.

Since the buffering solution employed in the process of this invention is acidic, it can also be used as the acidic solution reaction component. Here, an excess amount of the buffering solution would be introduced into reaction zone 39.

When practicing the embodiment of the invention wherein the iodine is derived from a reaction between an iodate, an iodide and an acidic solution, the flow rate and concentration of the iodate solution, the iodide solution, the iodate/iodide solution and/or the acidic solution depend, in part, upon many different variables. Examples of some variables which should be taken into consideration when determining flow rates and/or concentrations include, but are not limited to, flow rate of sample solution exiting constant level box 22 through stream 34, specific composition of acidic solution, pH of acidic solution, pH of iodate solution, pH of the iodide solution, time needed for the specific iodate, the specific iodide, and the specific acidic solution to react with one another and form an iodine-containing reaction mixture, the amount of iodine necessary for the specific biasing process, the normality of the iodine necessary for the specific biasing process, the amount and/or pH of the buffering solution necessary for the specific biasing process, and the like. After understanding this embodiment of the present invention, a skilled artisan should be able to take the above variables into consideration and determine the optimum flow rates and concentrations of the specific iodate, the specific iodide and the specific acidic solutions to be employed.

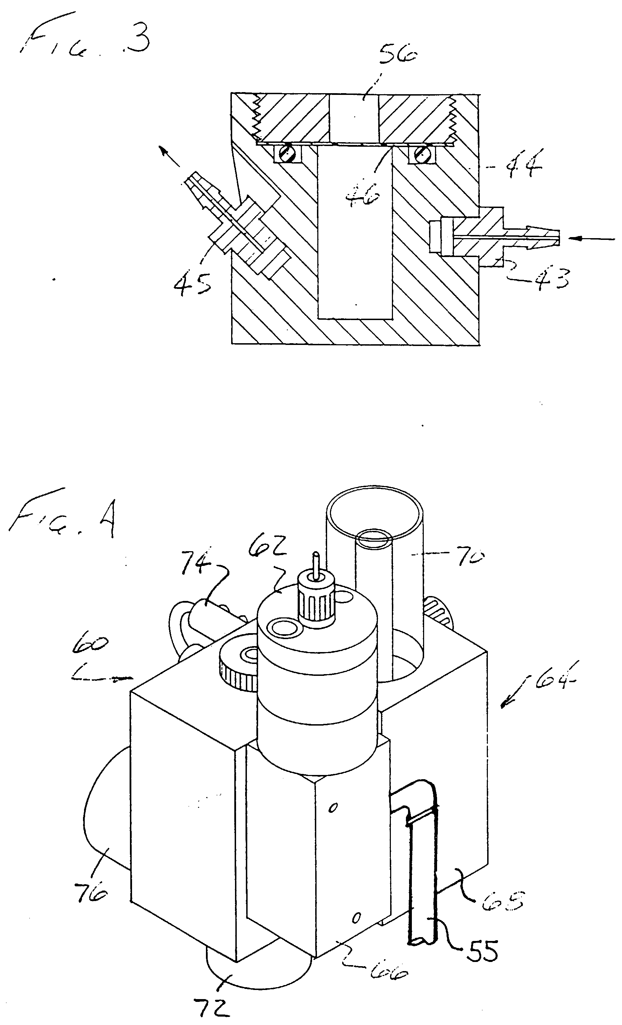

Referring now to Figure 3, the gas purger 44 of Figure 1 is illustrated in greater detail. Preferably, gas purger 44 is a sealed container of cylindrical construction although other shapes are permissible. Stream 36 enters purger 44 through inlet 43 and exits from outlet 45. Closely fitted within gas purger 44 is a hydrophobic membrane 46 which is permeable to the gas bubbles in stream 36. Membrane 46 allows any gas bubbles which are present within the iodine solution of stream 36 to be removed before the iodine solution is introduced into the dechlorinated process sample. The trapped gas bubbles are then exhausted to the atmosphere through vent 56. Gas purger 44 ensures that the iodine solution within stream 36 is void of gas bubbles so that the exact amount of iodine added to the dechlorinated process sample is known. Gas bubbles, which may be present in stream 36, will produce an inaccurate measure of the amount of iodine in stream 36. Ultimately, this condition will result in an inaccurate measurement for the amount of dechlorination residual within the process sample.

It should be noted that, although not illustrated, it is within the scope of this invention to employ gas purger 44 along stream 39 of Figure 2.

Referring now to Figure 4, the internal assembly of analyzer 52 is shown in greater detail. Residual analyzer assembly 60 comprises a probe portion 62 and a working fluid sampler system 64. The working fluid sampler system 64 includes a probe block 66 which supports the probe 62 and a flow block 65 which defines the flow passageways of the sampler system 64. Preferably, an amperometric type probe utilizing bare or exposed electrodes is selected. A constant level box 70 maintains the sample to be analyzed at a constant head. An orifice-cleaning mechanism 72, an adjustable flow delay 74, and an impeller mechanism chamber 76 also make up assembly 60. The various internal passageways direct a sample of the flow through fluid sampler system 64 for analyzation by probe 62 and then exhaust the flow for collection in drain 29, via stream 55.

The invention will still more fully be understood from the following examples. These examples are intended to illustrate embodiments of the invention wherein the iodine source is derived from a reaction between an iodate, an iodide and an acidic solution. It should be noted that these examples are, in no way, intended to limit the scope of this invention.

### Example I

This example demonstrates the preparation of iodate/iodide solutions useful as reaction components to produce an iodine-containing reaction product. Specifically, this example will demonstrate two methods in which to prepare an iodate/iodide solution such that, when the solution is subjected to an acidic environment, the resulting iodine solution produced will have a normality of 0.00679 N.

### Preferred Method

An iodate solution is prepared by dissolving 0.92 grams of potassium iodate in one-half gallon of distilled water at room temperature and standard pressure, while stirring for 15 minutes. An iodide solution is then prepared by dissolving 160 grams of potassium iodide and 6 grams of potassium hydroxide in one-half gallon of distilled water.

The iodate and iodide solutions are then mixed and stirred at room temperature and standard pressure to produce an iodate/iodide solution. The pH of the iodate/iodide solution is greater than 11.5.

When the iodate/iodide solution is mixed with acetic acid, the reaction components react with one another to produce, among other things, an iodine solution having a normality of 0.00679 N.

### Alternate Method

An alkaline solution is prepared by dissolving 10 grams of potassium hydroxide in 1 liter of distilled water. Thereafter, 160 grams of potassium iodide and 3.27 grams of iodine crystals are dissolved in the alkaline solution.

The reaction mixture is then permitted to stand for approximately 24 hours to produce an iodate/iodide solution. When the iodate/iodide solution is mixed with acetic acid, the reaction components react with one another to produce among other things, an iodine solution having a normality of 0.00679 N.

The conditions in the foregoing description are for illustration only and should not be construed as limiting the scope of the invention: the present invention may be embodied in other specific forms.

## Claims

1. A method for determining the amount of residual disinfectant removing agent in a continuous process stream to which a disinfectant has been added and said disinfectant removing agent has been added to completely remove residual disinfectant, comprising the steps of:

   (a) continuously drawing off a sample of said process stream;
   (b) continuously adding to said sample an analyzing agent different from said disinfectant, in an amount sufficient to completely react with residual disinfectant removing agent and leave an unreacted amount of analyzing agent;
   (c) allowing sufficient time for the analyzing agent to completely react with residual disinfectant removing agent;
   (d) continuously analyzing the sample to determine the amount of unreacted analyzing agent remaining in the sample;
   (e) continuously determining the amount of residual disinfectant removing agent in the sample based on the amount of analyzing agent added and the amount of unreacted analyzing agent remaining in the sample after reaction; and
   (f) using the determined amount of residual disinfectant removing agent to continuously selectably vary the amount of disinfectant removing agent added to the process stream.

2. A method according to claim 1, wherein the disinfectant is chlorine.

3. A method according to claim 1, wherein the residual disinfectant removing agent is sulphur dioxide.

4. A method according to any preceding claim, wherein the analyzing agent is selected from the group consisting of iodine and bromine.

5. A method according to claim 4, wherein the analyzing agent is iodine.

6. A method according to claim 5, wherein said iodine is at least partially supplied from an iodine source.

7. A method according to claim 5, wherein said iodine is a reaction product.

8. A method according to claim 7, wherein said iodine results from a chemical reaction between an iodate and an iodide when subjected to a pH less than about 9.5.

9. A method for determining the amount of residual dechlorination agent in a continuous process stream to which chlorine has been added and said dechlorination agent has been added to completely remove residual chlorine, comprising the steps of:

   (a) continuously drawing off a sample of said process stream;
   (b) continuously adding to said sample an iodine-containing analyzing agent, in an amount sufficient to completely react with residual dechlorination agent, and leave an unreacted amount of the iodine-containing analyzing agent;
   (c) allowing sufficient time for the iodine-containing analyzing agent to completely react with residual dechlorination agent;
   (d) continuously analyzing the sample to determine the amount of unreacted iodine-containing analyzing agent remaining in the sample;
   (e) continuously determining the amount of residual dechlorination agent in the sample based on the amount of iodine-containing analyzing agent added and the amount of unreacted iodine-containing analyzing agent remaining in the sample after reaction; and
   (f) using the determined amount of residual dechlorination agent to continuously selectably vary the amount of dechlorination agent added to the process stream.

10. A method according to claim 9, wherein the iodine containing analyzing agent comprises an iodine/iodide solution.

11. A method for determining the amount of residual sulfur dioxide in a continuous process stream to which chlorine has been added and said sulfur dioxide agent has been added to completely remove residual chlorine, comprising the steps of:

   (a) continuously drawing off a sample of said process stream;
   (b) continuously adding to said sample an iodine analyzing agent, in an amount sufficient to completely react with the residual sulfur dioxide and leave an unreacted amount of iodine analyzing agent;
   (c) allowing sufficient time for the iodine analyzing agent to completely react with residual sulfur dioxide;
   (d) continuously analyzing the sample to determine the amount of unreacted iodine analyzing agent remaining in the sample;
   (e) continuously determining the amount of residual sulfur dioxide in the sample based on the amount of iodine analyzing agent added and the amount of unreacted iodine analyzing agent remaining in the sample after reaction; and
   (f) using the determined amount of residual sulfur dioxide to continuously selectably vary the amount of sulfur dioxide added to the process stream.

12. A system for determining the amount of residual disinfectant removing agent in a continuous process stream (12) to which a disinfectant has been added and said disinfectant removing agent has been added to completely remove residual disinfectant, comprising:

   (a) means (20) for continuously drawing off a sample (18) of said process stream (12);
   (b) feed means for adding an analyzing agent (36) different from said disinfectant to said sample;
   (c) analyzer means (52) for analyzing the sample containing added analyzing agent to determine the amount of residual disinfectant removing agent in said sample; and
   (d) controller means (54) responsive to the analyzer means and the feed means for continuously selectably varying the amount of disinfectant removing agent added to the process stream based on the determined amount of residual disinfectant removing agent.

13. A system according to claim 12, further comprising means for purging gas from the analyzing agent added to said sample.

14. A system according to claim 13, wherein the means for purging gas from the analyzing agent comprises a hyrophobic membrane closely fitted within a sealed container.

15. A system according to claim 12, 13 or 14, further comprising means for adding the analyzing agent to said sample at a determinable rate.

**16.** A system according to any of claims 12 to 15, wherein the means for analyzing the sample to determine the amount of unreacted analyzing agent remaining in the sample is an amperometric analyzer.

## Patentansprüche

**1.** Verfahren zur Bestimmung der Menge an Entfernungsmittel für ein restliches Desinfektionsmittel in einem kontinuierlichen Prozesstrom, dem ein Desinfektionsmittel hinzugeführt wurde und bei dem das Entfernungsmittel für das Desinfektionsmittel hinzugegeben wurde, um Restbestände an Desinfektionsmittel vollständig zu beseitigen, gekennzeichnet durch die folgenden Schritte:

    (a) kontinuierliche Abnahme einer Probe aus dem Prozesstrom,

    (b) kontinuierliches Hinzugeben eines Analysemittel zur Probe, das sich vom Desinfektionsmittel unterscheidet, in einer ausreichenden Menge, damit das Analysemittel vollständig mit dem Entfernungsmittel für das restliche Desinfektionsmittel reagiert und eine nicht reagierte Menge an Analysemittel zurückbleibt,

    (c) Ermöglichung einer ausreichenden Zeit, damit das Analysemittel vollständig mit dem Entfernungsmittel für das restliche Desinfektionsmittel reagieren kann,

    (d) kontinuierliche Analyse der Probe, um die Menge an nicht reagiertem Analysemittel, welches in der Probe verbleibt, zu bestimmen,

    (e) kontinuierliche Bestimmung der Menge an Entfernungsmittel für das restliche Desinfektionsmittel in der Probe auf der Basis der hinzugefügten Menge an Analysemittel und der Menge an nicht reagiertem Analysemittel, das in der Probe nach der Reaktion verbleibt, und

    (f) Verwendung der bestimmten Menge an Entfernungsmittel für das restliche Desinfektionsmittel, um kontinuierlich gezielt die Menge an dem Prozesstrom hinzugefügtem Entfernungsmittel für das Desinfektionsmittel zu ändern.

**2.** Verfahren nach Anspruch 1, dadurch gekennzeichnet, dass das Desinfektionsmittel Chlor ist.

**3.** Verfahren nach Anspruch 1, dadurch gekennzeichnet, dass das Entfernungsmittel für das restliche Desinfektionsmittel Schwefeldioxid ist.

**4.** Verfahren nach einem der vorhergehenden Ansprüche, dadurch gekennzeichnet, dass das Analysemittel aus der Gruppe ausgewählt wird, die Jod und Brom umfasst.

**5.** Verfahren nach Anspruch 4, dadurch gekennzeichnet, dass das Analysemittel Jod ist.

**6.** Verfahren nach Anspruch 5, dadurch gekennzeichnet, dass das Jod wenigstens teilweise von einer Jodquelle geliefert wird.

**7.** Verfahren nach Anspruch 5, dadurch gekennzeichnet, dass das Jod ein Reaktionsprodukt ist.

**8.** Verfahren nach Anspruch 7, dadurch gekennzeichnet, dass das Jod aus einer chemischen Reaktion zwischen einem Jodat und einem Jodid stammt, wenn einem pH weniger etwa 9,5 unterworfen.

**9.** Verfahren zur Bestimmung der Menge an restlichem Entchlorungsmittel in einem kontinuierlichen Prozesstrom, dem Chlor hinzugefügt wurde und das Entchlorungsmittel hinzugegeben wurde, um vollständig Restbestände an Chlor zu entfernen, gekennzeichnet durch die Schritte:

    (a) kontinuierliches Abnehmen einer Probe aus dem Prozesstrom,

    (b) kontinuierliches Hinzufügen eines Jod enthaltenden Analysemittels zur Probe in einer ausreichenden Menge, um vollständig mit dem restlichen Entchlorungsmittel zu reagieren und eine nicht reagierte Menge an Jod enthaltendem Analysemittel zurückzulassen,

    (c) Ermöglichung einer ausreichenden Zeit für das Jod enthaltende Analysemittel, um vollständig mit dem restlichen Entchlorungsmittel zu reagieren,

    (d) kontinuierliches Analysieren der Probe zur Bestimmung der Menge an nicht reagiertem Jod enthaltendem Analysemittel, die in der Probe verblieben ist,

    (e) kontinuierliche Bestimmung der Menge an restlichem Entchlorungsmittel in der Probe auf der Basis der Menge an hinzugefügtem Jod enthaltenden Analysemittel und der Menge an nicht reagiertem Jod enthaltenden Analysemittel, welche in der Probe nach der Reaktion verblieben ist, und

    (f) Verwendung der bestimmten Menge an restlichem Entchlorungsmittel zur kontinuierlichen gezielten Änderung der Menge an dem Prozesstrom hinzugefügtem Entchlorungsmittel.

10. Verfahren nach Anspruch 9, dadurch gekennzeichnet, dass das Jod enthaltende Analysemittel eine Jod/Jodidlösung umfasst.

11. Verfahren zur Bestimmung der Menge an restlichem Schwefeldioxid in einem kontinuierlichen Prozesstrom, dem Chlor hinzugefügt worden ist und das Schwefeldioxidmittel hingefügt wurde, um restliches Chlor vollständig zu entfernen, gekennzeichnet durch die Schritte:

(a) kontinuierliches Abnehmen einer Probe aus dem Prozesstrom,

(b) kontinuierliches Hinzufügen zur Probe eines Jodanalysemittels in einer ausreichenden Menge, um vollständig mit dem restlichen Schwefeldioxid zu reagieren und eine nicht reagierte Menge an Jodanalysemittel zurückzulassen,

(c) Ermöglichung einer ausreichenden Zeit für das Jodanalysemittel, um vollständig mit dem restlichen Schwefeldioxid zu reagieren,

(d) kontinuierliches Analysieren der Probe zur Bestimmung der Menge an nicht reagiertem Jodanalysemittel, das in der Probe verblieben ist,

(e) kontinuierliche Bestimmung der Menge an restlichem Schwefeldioxid in der Probe auf der Basis der Menge an hinzugefügtem Jodanalysemittel und der Menge an nicht reagiertem Jodanalysemittel, die in der Probe nach der Reaktion verblieben ist, und

(f) Verwendung der bestimmten Menge an restlichem Schwefeldioxid zur kontinuierlichen gezielten Änderung der Menge an dem Prozesstrom hinzugefügtem Schwefeldioxid.

12. System zur Bestimmung der Menge an Entfernungsmittel für ein restliches Desinfektionsmittel in einem kontinuierlichen Prozesstrom (12), dem ein Desinfektionsmittel hinzugefügt worden ist und das Entfernungsmittel für das Desinfektionsmittel hinzugegeben wurde, um vollständig das restliche Desinfektionsmittel zu entfernen, gekennzeichnet durch

(a) eine Einrichtung (20) zum kontinuierlichen Abnehmen einer Probe (18) aus dem Prozesstrom (12),

(b) eine Zuführeinrichtung zum Zugeben eines Analysemittels (36) zur Probe, das sich vom Desinfektionsmittel unterscheidet,

(c) eine Analyseinrichtung (52) zur Analyse der Probe, die das hinzugebene Analysemittel enthält, um die Menge an Entfernungsmittel für das restliche Desinfektionsmittel in der Probe zu bestimmen, und

(d) eine Steuereinrichtung (54), die in Abhängigkeit von der Analyseeinrichtung und der Zuführeinrichtung kontinuierlich gezielt die Menge an dem Prozesstrom hinzugefügtem Entfernungsmittel für das Desinfektionsmittel auf der Basis der bestimmten Menge an Entfernungsmittel für das restliche Desinfektionsmittel ändert.

13. System nach Anspruch 12, ferner gekennzeichnet durch eine Einrichtung zur Reinigung von Gas aus dem der Probe hinzugegebenen Analysemittel.

14. System nach Anspruch 13, dadurch gekennzeichnet, dass die Einrichtung zur Reinigung von Gas aus dem Analysemittel eine hydrophobe Membran umfasst, die in einem abgedichteten Behälter abgeschlossen gehalten ist.

15. System nach Anspruch 12, 13 oder 14, ferner gekennzeichnet durch eine Einrichtung zur Zufügung des Analysemittels zur Probe mit einer bestimmbaren Rate.

16. System nach einem der Ansprüche 12 bis 15, dadurch gekennzeichnet, dass die Einrichtung zum Analysen der Probe zur Bestimmung der Menge an nicht reagiertem in der Probe verbleibenden Analysemittel ein amperometrisches Analysegerät ist.

**Revendications**

1. Procédé pour déterminer la quantité d'agent d'élimination du désinfectant résiduel dans un courant d'un procédé continu auquel un désinfectant a été ajouté et ledit agent d'élimination du désinfectant a été ajouté afin d'éliminer complètement le désinfectant résiduel, comprenant les étapes consistant:

(a) à prélever d'une manière continue un échantillon dudit courant;
(b) à ajouter d'une manière continue audit échantillon un agent d'analyse différent dudit désinfectant, dans une proportion suffisante pour qu'il réagisse complètement avec l'agent d'élimination du désinfectant résiduel et pour laisser une quantité d'agent d'analyse n'ayant pas réagi;

(c) à laisser suffisamment de temps pour que l'agent d'analyse réagisse complètement avec l'agent d'élimination du désinfectant résiduel;

(d) à analyser d'une manière continue l'échantillon pour déterminer la quantité d'agent d'analyse n'ayant pas réagi qui reste dans l'échantillon;

(e) à déterminer d'une manière continue la quantité d'agent d'élimination du désinfectant résiduel dans l'échantillon, sur la base de la quantité d'agent d'analyse ajoutée et de la quantité d'agent d'analyse n'ayant pas réagi qui reste dans l'échantillon après la réaction; et

(f) à utiliser la quantité déterminée d'agent d'élimination du désinfectant résiduel pour faire varier d'une manière continue et sélective la quantité d'agent d'élimination du désinfectant ajoutée au courant.

2. Procédé selon la revendication 1, dans lequel le désinfectant est le chlore.

3. Procédé selon la revendication 1, dans lequel l'agent d'élimination du désinfectant résiduel est l'anhydride sulfureux.

4. Procédé selon l'une quelconque des revendications 1 à 3, dans lequel l'agent d'analyse est choisi dans le groupe constitué par l'iode et le brome.

5. Procédé selon la revendication 4, dans lequel l'agent d'analyse est l'iode.

6. Procédé selon la revendication 5, dans lequel ledit iode est fourni au moins partiellement par une source d'iode.

7. Procédé selon la revendication 5, dans lequel ledit iode est un produit de réaction.

8. Procédé selon la revendication 7, dans lequel ledit iode résulte d'une réaction chimique entre un iodate et un iodure lorsqu'ils sont soumis à un pH inférieur à 9,5 environ.

9. Procédé pour déterminer la quantité d'agent de déchloration résiduel dans un courant d'un procédé continu auquel du chlore a été ajouté et ledit agent de déchloration a été ajouté afin d'éliminer complètement le chlore résiduel, comprenant les étapes consistant:

(a) à prélever d'une manière continue un échantillon dudit courant;

(b) à ajouter d'une manière continue audit échantillon un agent d'analyse contenant de l'iode, dans une proportion suffisante pour qu'il réagisse complètement avec l'agent de déchloration résiduel et pour laisser une quantité d'agent d'analyse contenant de l'iode n'ayant pas réagi;

(c) à laisser suffisamment de temps pour que l'agent d'analyse contenant de l'iode réagisse complètement avec l'agent de déchloration résiduel;

(d) à analyser d'une manière continue l'échantillon pour déterminer la quantité d'agent d'analyse contenant de l'iode n'ayant pas réagi qui reste dans l'échantillon;

(e) à déterminer d'une manière continue la quantité d'agent de déchloration résiduel dans l'échantillon, sur la base de la quantité d'agent d'analyse contenant de l'iode ajoutée et de la quantité d'agent d'analyse contenant de l'iode n'ayant pas réagi qui reste dans l'échantillon après la réaction; et

(f) à utiliser la quantité déterminée d'agent de déchloration résiduel pour faire varier d'une manière continue et sélective la quantité d'agent de déchloration ajoutée au courant de traitement.

10. Procédé selon la revendication 9, dans lequel l'agent d'analyse contenant de l'iode comprend une solution d'iode/iodure.

11. Procédé pour déterminer la quantité d'anhydride sulfureux résiduel dans un courant d'un procédé continu auquel du chlore a été ajouté et ledit anhydride sulfureux a été ajouté afin d'éliminer complètement le chlore résiduel, comprenant les étapes consistant:

(a) à prélever d'une manière continue un échantillon dudit courant;

(b) à ajouter d'une manière continue audit échantillon un agent d'analyse à l'iode, dans une proportion suffisante pour qu'il réagisse complètement avec l'anhydride sulfureux résiduel et pour laisser une quantité d'agent d'analyse à l'iode n'ayant pas réagi;

(c) à laisser suffisamment de temps pour que l'agent d'analyse à l'iode réagisse complètement avec l'anhydride sulfureux résiduel;

(d) à analyser d'une manière continue l'échantillon pour déterminer la quantité d'agent d'analyse à l'iode n'ayant pas réagi qui reste dans l'échantillon;

(e) à déterminer d'une manière continue la quantité d'anhydride sulfureux résiduel dans l'échantillon, sur la base de la quantité d'agent d'analyse à l'iode ajoutée et de la quantité d'agent d'analyse à l'iode n'ayant pas réagi qui reste dans l'échantillon après la réaction; et

(f) à utiliser la quantité déterminée d'anhydride sulfureux résiduel pour faire varier d'une manière continue et sélective la quantité d'anhydride sulfureux ajoutée au courant de traitement.

12. Système pour déterminer la quantité d'agent d'élimination du désinfectant résiduel dans un courant d'un procédé continu (12) auquel un désinfectant a été ajouté et ledit agent d'élimination du désinfectant a été ajouté afin d'éliminer complètement le désinfectant résiduel, comprenant:

> (a) un moyen (20) pour prélever d'une manière continue un échantillon (18) dudit courant de traitement (12);
> (b) un moyen d'alimentation pour ajouter audit échantillon un agent d'analyse (36) différent dudit désinfectant;
> (c) un moyen analyseur (54) pour analyser l'échantillon contenant l'agent d'analyse ajouté, afin de déterminer la quantité d'agent d'élimination du désinfectant résiduel dans ledit échantillon; et
> (d) un moyen de commande (54) qui réagit au moyen analyseur et au moyen d'alimentation en faisant varier d'une manière continue et sélective la quantité d'agent d'élimination du désinfectant qui est ajoutée au courant, sur la base de la quantité déterminée d'agent d'élimination du désinfectant résiduel.

13. Système selon la revendication 12, comprenant en outre un moyen pour purger des gaz l'agent d'analyse ajouté audit échantillon.

14. Système selon la revendication 13, dans lequel le moyen pour purger des gaz l'agent d'analyse comprend une membrane hydrophobe fixée de façon étanche dans un récipient fermé.

15. Système selon l'une quelconque des revendications 12 à 14, comprenant en outre un moyen pour ajouter l'agent d'analyse à l'échantillon dans une mesure déterminable.

16. Système selon l'une quelconque des revendications 12 à 15, dans lequel le moyen d'analyse de l'échantillon pour déterminer la quantité d'agent d'analyse n'ayant pas réagi qui reste dans l'échantillon est un analyseur ampérométrique.

FIG. 1

FIG. 2

Fig. 3

56

44

46

45

43

Fig. A

74

62

70

60

64

76

65

55

72

66